# EUROPÄISCHE PATENTANMELDUNG

(11) **EP 0 655 237 A1**
(43) Veröffentlichungstag der Anmeldung: **31.05.1995**
(21) Anmeldenummer: 94118290.9
(22) Anmeldetag: 21.11.1994
(51) Int. Cl.: A61K 9/00

(54) **Medizinische Aerosolformulierung**

(30) Priorität: 27.11.1993 DE 4340434
(71) Anmelder: HOECHST AKTIENGESELLSCHAFT, D-65929 Frankfurt am Main (DE)
(72) Erfinder: Ditzinger, Günter, Dr., D-65929 Frankfurt (DE); Zott, Wolfgang, D-65795 Hattersheim (DE)

(57) **Zusammenfassung**

Die Erfindung betrifft eine Arzneistoffzubereitung in Form eines Suspensionsaerosols, die ein sprühgetrocknetes Produkt, welches aus einem Arzneistoff, einer im verflüssigten Treibmitte unlöslichen, oberflächenaktiven, physiologisch verträglichen Substanz, gegebenenfalls ein Geschmackskorrigens und/oder einen üblichen Hilfsstoff, in einem verflüssigbaren, hydrierten oder teilhydrierten Fluorkohlenwasserstoffs als Treibmittel, enthält. Ferner betrifft die Erfindung ein Verfahren zur Arzneistoffzubereitung, die lokal auf der Lunge wirksame, zur Inhalation geeignete Arzneistoffe zur Behandlung von Atemwegserkrankungen oder Asthma enthält.

## Beschreibung

Die moderne Aerosol-Technik, die sich überwiegend auf die Verwendung Von unter mäßigem Druck verflüssigbaren Sicherheitstreibmitteln stützt, bringt eine Reihe bedeutender Vorteile und hat darüber hinaus zahlreiche neue Anwendungsmöglichkeiten erschlossen. Im einzelnen sind folgende Vorteile hervorzuheben:
Jede Druckgaspackung ist ein Automat, der es gestattet, durch Fingerdruck auf einen Applikator das Produkt in der für eine optimale Wirkung geeigneten Form zu entnehmen bzw. aufzutragen. Mit dieser Steuerung läßt sich die Menge des anzuwendenden Produktes leicht und somit auch rationell einteilen. Wo Entnahme in immer gleicher und kleiner Dosis angebracht ist, übernimmt ein Dosierventil diese Mengenbegrenzung automatisch.

Die Bequemlichkeit der Handhabung solcher Druckgaspackungen ist ein entscheidender Vorteil dieser Arzneiform. Die Einhand-Geräte sind praktisch im Gebrauch und einfach in der Handhabung. Durch das selbständig schließende Ventil kann der Inhalt nicht ausfließen oder verschüttet werden. Flüchtige Stoffe können nicht verdunsten, und der Inhalt kann nicht austrocknen. Der gasdichte Abschluß der Packung verhindert den Zutritt von Luft und die damit möglichen Verunreinigungen durch Staub, Feuchtigkeit oder Keime. Oxydationsempfindliche Produkte können unter Ausschluß von Luftsauerstoff abgefüllt werden. Eine Druckgaspakkung bietet auch einen hervorragenden Lichtschutz für empfindliche Wirkstoffe.

Das Füllprodukt der Druckgaspackungen besteht häufig aus einer pulverförmigen Substanz (z.B. einem Arzneistoff), die im verflüssigten Treibmittel suspendiert vorliegt, sowie einer oberflächen- aktiven Substanz, die zur Stabilisierung der Suspension dient, z.B. Sorbitantrioleat, Ölsäure oder Lecithin. Als Treibmittel für medizinische Aerosole wurden bisher fast ausschließlich Fluorchlorkohlenwasserstoffe eingesetzt, z.B. Trichlorfluormethan (@Frigen 11), Dichlordifluormethan (Frigen 12), 1,2-Dichlortetrafluorethan (Frigen 114), sowie deren Gemische. Das Pulver, das eine möglichst geringe Teilchengröße haben muß, um einer Sedimentation vorzubeugen, wird in einer wesentlich größeren Treibmittelmenge dispergiert. Der Treibmittelmengenanteil muß auch zur Verminderung der Gefahr von Ventilstörungen mindestens 85 Gew.-% betragen und wird in vielen Fällen erheblich über diesem Wert liegen. Solche Aerosole finden wir häufig als Inhalationsaerosole wieder.

Inhalationsaerosole sind geeignet zur Verabreichung von Arzneistoffen zur Therapie von Erkrankungen der Atemwege, z.B. der Verabreichung von Beta-Sympathomimetika, Steroiden, Anticholinergika, Antihistaminika, Mastzellstabilisatoren, wie Cromoglicinsäure oder Nedocromil, PAF-Antagonisten, Leukotrienantagonisten, Bradykininantagonisten oder Kaliumkanal-aktivatoren zur lokalen Therapie von Asthma. Inhalationsaerosole sind auch zur systemischen Therapie von Krankheiten geeignet, da das Lungenepithel eine ausreichende Permeabilität für niedermolekulare Arzneistoffe besitzt. Die pulmonale Applikation mittels Inhalationsaerosol eignet sich vor allem für hochwirksame Arzneistoffe, z.B. Peptide und Proteine wie Insulin, LHRH-Analoga, Oxytocin, Vasopressin-Analoga, Calcitonin-Analoga oder Interferon (vergl. z.B. Banga und Chien, Int. J. Pharm. 48, 15-50 (1988)). Suspensionsaerosolformulierungen von LHRH-Analoga mit Fluorchlorkohlenwasserstoff als Treibmittel sind z.B. in EP-A-0 510 731 beschrieben.

Diskussionen über die Ursache der Schädigung der Ozonschicht durch Fluorchlorkohlenwasserstoffe (FCKW) haben dazu geführt, daß die Verwendung dieser Stoffe in vielen Ländern eingeschränkt oder teilweise sogar verboten wurde. Aus Untersuchungen ist bekannt, daß eine der Ursachen, die zur Schädigung der Ozonschicht führen, die Reaktion von Ozon mit aus Chloratomen der FCKW's entstehenden Radikalen ist. Daher werden in jüngster Zeit für Aerosole nicht-ozonschädigende Treibmittel z.B. Kohlendioxid, Distickstoffoxid, Dimethylether, kurzkettige Kohlenwasserstoffe (Propan oder Butan) oder hydrierte Fluorkohlenwasserstoffe (HFKW) eingesetzt.

Als Treibmittel für pharmazeutische Aerosole eignen sich insbesondere solche Treibgase, welche sich unter Druck bei Raumtemperatur verflüssigen lassen und bei Inhalationen oder topischer Anwendung sicher, toxikologisch unbedenklich und Nebenwirkungs-frei sind. Diese Eigenschaften treffen vor allem auf hydrierte Fluorkohlenwasserstoffe (HFKW), wie z.B. Tetrafluorethan (R134a) und Heptafluorpropan (R227) zu, wobei Heptafluorpropan aufgrund seines niedrigeren Dampfdruckes bei Raumtemperatur von ca. 4 bar besser geeignet ist, da auf einen druckvermindernden Zusatz verzichtet werden kann. Tetrafluorethan kann aufgrund seines höheren Dampfdruckes von 6 bar bei Raumtemperatur als alleiniges Treibmittel nicht eingesetzt werden, da es bei 50 _{°} C den nach TRG300 (Technische Richtlinien Gase) zulässigen Höchstdruck für Aluminiumdosen von 12 bar mit einem Druck von ca. 13 bar überschreitet.

Die bisher verwendete Technologie zur Herstellung von medizinischen Suspensionsaerosolen beruht auf einer Löslichkeit der oberflächenaktiven Substanzen im verflüssigten Treibmittel (der Arzneistoff ist im Treibmittel suspendiert). Diese Eigenschaft ist bei Verwendung von neuen, alternativen Treibmitteln, wie Tetrafluorethan oder Heptafluorpropan, aufgrund deren höherer Polarität nicht mehr gegeben. Dies bedeutet, daß Formulierungen mit Fluorchlorkohlenwasserstoffen nicht einfach nur durch Austausch des Treibmittels gegen hydrierte Fluorkohlenwasserstoffe umgestellt werden können, wie dies in EP-A-0 513 099, EP-A-0 518 600, EP-A-0 518 601 und EP-A-0 550 031 beschrieben wird. Auf diese Weise können mit den bisher in Inhalationsaerosolen verwendeten oberflächenaktiven Substanzen keine stabilen Suspensionen hergestellt werden.

Gemäß EP-B-0 372 777 und EP-A-0 499 344 werden daher Kosolventien mit einer höheren Polarität wie z.B. Ethanol verwendet, um eine ausreichende Löslichkeit der oberflächenaktiven Substanz im verflüssigten Treibmittel (HFKW) zu erreichen. Die Verwendung von anderen, im verflüssigten Treibmittel (HFKW) löslichen, jedoch bisher in medizinischen Aerosolen nicht verwendeten oberflächenaktiven Substanzen wird in EP-A-0 504 112 (monoacetylierte oder diacetylierte Monoglyceride), EP-A-0 536 204, EP-A-0 513 127 und EP-A-526 481 (fluorierte Tenside), EP-A-0 536 235 (Blockcopolymere aus Ethylenoxid und Propylenoxid sowie Polysorbate) und EP-A-0 534 731 (Polyvinylpyrrolidon und Polyvinylalkohol)beschrieben. Die genannten Stoffe besitzen jedoch den entscheidenden Nachteil, daß sie für eine inhalative Anwendung nicht toxikologisch geprüft, oder sogar erwiesenermaßen ungeeignet, da gewebeschädigend, sind.

Weiterhin ist in den Patentanmeldungen WO 92/08446 und WO 92/08447 ein Verfahren beschrieben, bei dem Wirkstoffe mit der oberflächen- aktiven Substanz überzogen werden. Dabei wird von einem bereits mikronisierten Wirkstoff ausgegangen, der in einer Lösung der oberflächenaktiven Substanz in einem organischen Lösungsmittel, wie z.B. Isopentan, in dem der Wirkstoff praktisch unlöslich ist, suspendiert wird. Nach einer gewissen Zeit wird das Lösungsmittel entfernt. Die so modifizierten Wirkstoffe lassen sich in hydrierten Fluorkohlenwasserstoffen (HFKW) suspendieren, gegebenenfalls unter Zugabe eines Kosolvens. Ein Nachteil dieses Verfahrens ist, daß der zuvor mikronisierte Wirkstoff suspendiert und wieder getrocknet werden muß. Dabei kann es zu Agglomerationen der Partikel kommen. Das Verfahren gewährleistet keine gleichmäßige Verteilung der oberflächenaktiven Substanz. Auch die Verwendung eines zusätzlichen organischen Lösungsmittels wie Isopentan ist negativ zu beurteilen.

Der Erfindung lag nun die Aufgabe zugrunde, ein stabiles Suspensions-Dosieraerosol für medizinische Anwendung zu formulieren, enthaltend einen pharmazeutischen Wirkstoff, eine im verflüssigten Treibmittel unlösliche physiologisch verträgliche, oberflächenaktive Substanz, z.B. Sorbitantrioleat, Ölsäure und Lecithin, gegebenenfalls ein Geschmackskorrigens, z.B. einen Süßstoff wie Saccharin, Acesulfam K oder Aspartam, oder ein ätherisches Öl, gegebenenfalls einen allgemein üblichen Hilfsstoff aus der Gruppe der Zucker oder Zuckeralkohole, wie Laktose, Glucose oder Mannit, sowie als Treibmittel einen hydrierten Fluorkohlenwasserstoff, bevorzugt Heptafluorpropan (R227).

Die Aufgabe wird erfindungsgemäß gelöst, indem Wirkstoff und oberflächenaktive Substanz, gegebenenfalls mit den genannten zusätzlichen Hilfsstoffen durch Sprühtrocknung in eine Form überführt werden, in der sie miteinander in einer Matrix fein verteilt vorliegen. Überraschenderweise wurde gefunden, daß dieses sprühgetrocknete Produkt anschließend ohne weitere Zusätze im verflüssigten Treibmittel eine feine, stabile, homogene Suspension bildet.

Die Erfindung betrifft daher eine Arzneistoffzubereitung in Form eines Suspensionsaerosols, dadurch gekennzeichnet, daß sie
a) eine sprühgetrocknetes Produkt bestehend aus einem Arzneistoff und einer im verflüssigten Treibmittel unlöslichen, physiologisch verträglichen, oberflächenaktiven Substanz und gegebenenfalls einem Geschmackskorrigens und gegebenenfalls einem allgemein üblichen, physiologisch verträglichen Hilfsstoff und
b) einen verflüssigbaren, hydrierten oder teilhydrierten Fluorkohlenwasserstoff als Treibmittel enthält.

In dem sprühgetrockneten Produkt liegen Wirk-und Hilfsstoffe (einschließlich oberflächenaktive Substanzen) in einer Matrix fein verteilt vor. Nach Zusatz des Treibmittels entsteht eine feine, stabile, homogene Suspension. Die Produktteilchen weisen eine Partikelgröße auf, die für Aerosole üblich ist.

Die Erfindung betrifft ferner ein Verfahren zur Herstellung einer Arzneistoffzubereitung in Form eines Suspensionsaerosols, dadurch gekennzeichnet, daß man den Arzneistoff, die physiologisch verträgliche, oberflächenaktive Substanz und gegebenenfalls ein Geschmackskorrigens und gegebenenfalls weitere, allgemein übliche, physiologisch verträgliche Hilfsstoffe in einem geeigneten Lösungsmittel löst, die erhaltene Lösung einer Sprühtrocknung unterwirft, das sprühgetrocknete Produkt in eine Druckgaspackung eindosiert, diese mit einem Dosierventil verschließt und die zur Bildung des Suspensionsaerosol erforderliche Menge des verflüssigbaren, hydrierten oder teilhydrierten Fluorkohlenwasserstoffs zudosiert.

Als Arzneistoffe kommen solche in Betracht, die lokal auf der Lunge wirksam sind und sich zur Inhalation eignen, also z.B. Arzneistoffe zur Behandlung von Atemwegserkrankungen oder Asthma, wie Beta-Sympathomimetika, Steroide, Anticholinergika, Antihistaminika, Antiallergika, Mastzellstabilisatoren, wie Cromoglicinsäure oder Nedocromil, PAF-Antagonisten, Leukotrienantagonisten, Bradykininantagonisten oder Kaliumkanal-aktivatoren.

Die Sprühtrocknung eignet sich auch für pharmazeutische Wirkstoffe, die nicht mit konventionellen Methoden wie Mahlung mikronisiert und damit in eine für eine Inhalation nötige Teilchengröße überführt werden können. Dies gilt besonders für Peptide und Proteine, die durch Gefriertrocknung gewonnen werden und in amorpher Form vorliegen.

Als Wirkstoffe kommen daher auch Peptide und Proteine natürlichen oder synthetischen Ursprungs und deren physiologisch verträgliche Salze in Betracht. Als Beispiele seien genannt: Insuline, LHRH-Analoga, Oxytocin, Vasopressin-Analoga, Calcitonin-Analoga und Interferon und deren physiologisch verträgliche Salze.

Die erfindungsgemäßen Suspensionsaerosole enthalten als Arzneistoff vorzugsweise ein Insulin, einen Bradykininantagonisten, ein LHRH-Analogon wie Buserelin oder deren physiologisch verträgliche Salze.

Die erfindungsgemäßen Suspensionsaerosole eignen sich insbesondere zur Asthmabehandlung und enthalten z.B. den Bradykininantagonisten Icatibant (= H-D-Arg-Arg-Pro-Hyp-Gly-Thi-Ser-D-Tic-Oic-Arg-OH(HOE 140)) oder ein Icatibant-Salz oder den Kaliumkanal-Aktivator Rilmakalim ((+)-(3S,4R)-3-Hydroxy-2,2-dimethyl-4-(2-oxo-1-pyrrolidinyl)-6-phenyl-sulfonylchroman Hemihydrat).

Als oberflächenaktive Substanzen sind vor allem Sorbitantrioleat, Ölsäure oder Lecithin bevorzugt. Als Lecithin eignen sich alle natürlichen Lecithine, wie Ei- oder Sojalecithin, teilhydrierte und hydrierte Lecithine, und auch hochgereinigte Phosphatidylcholine.

Geeignete Geschmackskorrigentien sind z.B. Süßstoffe, wie Saccharin, Aspartam und Acesulfam K, oder ätherische Öle.

Die allgemein üblichen, physiologisch verträglichen Hilfsstoffe müssen, ebenso wie die Wirkstoffe, oberflächenaktiven Substanzen und Geschmackskorrigentien in dem gemeinsamen Lösungsmittel löslich sein. Die Natur dieser Hilfsstoffe richtet sich daher nach dem verwendeten Lösungsmittel. Besonders geeignet sind Hilfsstoffe aus der Gruppe der Zucker und Zuckeralkohole wie Laktose, Glucose oder Mannit.

Das sprühgetrocknete Produkt enthält einen oder mehrere Wirkstoffe, eine oder mehrere oberflächenaktive Substanzen und gegebenenfalls einen oder mehrere weitere Hilfsstoffe und Geschmackskorrigentien.

Als Treibmittel eignen sich z.B. Heptafluorpropan (R227) und Tetrafluorethan (R134a), vorzugsweise im Gemisch mit R227.

Als gemeinsames Lösungsmittel für die Bestandteile des sprühgetrockneten Produktes eignen sich z.B. Gemische aus niederen Alkoholen (mit bis zu 6 C-Atomen) oder Ketonen mit Wasser.

Der Wirkstoffanteil im sprühgetrockneten Produkt richtet sich insbesondere nach der Höhe der gewünschten Dosierung.

Der Anteil an oberflächenaktiver Substanz, die in der Wirkstoffmatrix verteilt ist, ist relativ gering und beträgt z.B. 0,01 bis 1,0 Gew.-%, vorzugsweise 0,05 bis 0,5 Gew.-%, bezogen auf den Wirkstoffanteil.

Die Sprühtrocknung wird nach üblichen Methoden, wie sie in der Literatur beschrieben sind (vergl. z.B. J. Broadhead et al., Drug Development and Industrial Pharmacy, 18, 1169-1206 (1992)) durchgeführt. Vorzugsweise wird sie bei erhöhter Temperatur durchgeführt, wobei die Höhe u.a. abhängig ist vom eingesetzten Wirkstoff und Lösungsmittel.

Durch das Verfahren der Sprühtrocknung bekommen die Wirkstoffteilchen eine sphärische, aerodynamische Form, wodurch die Bewegung im Luftstrom während des Einatmens begünstigt und dadurch der Anteil an lungengängigen Partikeln erhöht wird. Darüberhinaus werden Adhäsions- und Agglomerationskräfte sowohl in der Suspension als auch während des Aussprühvorgangs verringert. Das Verfahren der Sprühtrocknung zur Herstellung von Pulvern zu inhalativen Anwendung ist bereits aus den Patentschriften GB 1 520 248 und GB 1 569 612 bekannt. Hier wurden jedoch nur die reinen Wirkstoffe sprühgetrocknet ohne Zusatz von oberflächen-aktiven Substanzen, Geschmackskorrigentien oder weiteren Hilfsstoffen, und anschließend in Form eines Pulverinhalates und nicht als Suspensionsaerosol formuliert.

Die Erfindung wird anhand folgender Beispiele erläutert:

### Beispiel 1

1968 mg Icatibantacetat, 2,0 mg Soja-Lecithin S100 (Lipoid K.G.) und 30 mg Saccharin werden in einem Ethanol/Wasser-Gemisch (25 % G/G) klar gelöst und bei 110_{°}C unter inerten Bedingungen (N₂) in einer Sprühtrocknungsapparatur sprühgetrocknet. Das Produkt fällt dabei als feines, weißes Pulver an. Das Pulver wird in eine für Dosieraerosole vorgeschriebene Alumonobloc-Dose zu je 10 mg eindosiert, die Dose wird mit einem Dosierventil verschlossen und anschließend mit 10 g R 227 über das Dosierventil befüllt. Nach der Füllung entsteht sofort eine feine homogene Suspension primärer Arzneistoffteilchen in R 227. Ein Sprühstoß des nach dem Verfahren hergestellten Inhalationsaerosols (Suspensionsaerosols) enthält pro Applikation 100 ul Suspension, enthaltend 100 ug Arzneistoff.

### Beispiel 2

430 mg Icatibantacetat, 3566 mg Lactose und 4 mg hydriertes Ei-Lecithin (EPC-3, Lipoid K.G.) werden in einem Ethanol/Wasser-Gemisch (25 % G/G) klar gelöst und bei 90 _{°} C unter inerten Bedingungen (N₂) in einer Sprühtrocknungsapparatur sprühgetrocknet. Das Produkt fällt dabei als feines, weißes Pulver an. Das Pulver wird in eine für Dosieraerosole vorgeschriebene Alumonobloc-Dose zu je 10 mg eindosiert, die Dose wird mit einem Dosierventil verschlossen und anschließend mit 10 g R 227 über das Dosierventil befüllt. Nach der Füllung entsteht sofort eine feine homogene Suspension primärer Arzneistoffteilchen in R 227. Ein Sprühstoß des nach dem Verfahren hergestellten Inhalationsaerosols (Suspensionsaerosols) enthält pro Applikation 100 µl Suspension, enthaltend 10 ug Arzneistoff.

### Beispiel 3

1968 mg Icatibantacetat, 2 mg Sorbitantrioleat und 30 mg Aspartam werden in einem Ethanol/Wasser-Gemisch (25 % G/G) klar gelöst und bei 110°C unter inerten Bedingungen (N₂) in einer Sprühtrocknungsapparatur sprühgetrocknet. Das Produkt fällt dabei als feines, weißes Pulver an. Das Pulver wird in eine für Dosieraerosole vorgeschriebene Alumonobloc-Dose zu je 10 mg eindosiert, die Dose wird mit einem Dosierventil verschlossen und abschließend über das Dosierventil mit 10 g R 227 befüllt. Nach der Füllung entsteht sofort eine feine, homogene Suspension primärer Arzneistoffteilchen in R 227. Ein Sprühstoß des nach dem Verfahren hergestellten Inhalationsaerosols (Suspensionsaerosols) enthält pro Applikation 100 µl Suspension, enthaltend 100 µg Arzneistoff.

### Beispiel 4

1000 mg Humaninsulin, 2 mg Soja-Lecithin S100 und 1000 mg Laktose werden in einem Ethanol/Wasser-Gemisch (25 % G/G) klar gelöst und bei 90 ° C unter inerten Bedingungen (N₂) in einer Sprühtrocknungsapparatur sprühgetrocknet. Das Produkt fällt dabei als feines, weißes Pulver an. Das Pulver wird in eine für Dosieraerosole vorgeschriebene Alumonobloc-Dose zu je 50 mg eindosiert, die Dose wird mit einem Dosierventil verschlossen und abschließend über das Dosierventil mit 10 g R 227 befüllt. Nach der Füllung entsteht sofort eine feine homogene Suspension primärer Arzneistoffteilchen in R 227. Ein Sprühstoß des nach dem Verfahren hergestellten Inhalationsaerosols (Suspensionsaerosols) enthält pro Applikation 100 µl Suspension, entsprechend 31.U. Insulin.

### Beispiel 5

1998 mg Buserelin-Acetat und 2 mg Soja-Lecithin S100 werden in einem Ethanol/Wasser-Gemisch (25 % G/G) klar gelöst und bei 90 ° C unter inerten Bedingungen (N₂) in einer Sprühtrocknungsapparatur sprühgetrocknet. Das Produkt fällt dabei als feines, weißes Pulver an. Das Pulver wird in eine für Dosieraerosole vorgeschriebene Alumonobloc-Dose zu je 10 mg eindosiert, die Dose wird mit einem Dosierventil verschlossen und abschließend über das Dosierventil mit 10 g R 227 befüllt. Nach der Füllung entsteht sofort eine feine, homogene Suspension primärer Arzneistoffteilchen in R 227. Ein Sprühstoß des nach diesem Verfahren hergestellten Inhalationsaerosols (Suspensionsaerosols) enthält pro Applikation 100 µl Suspension, enthaltend 100 µg Arzneistoff.

### Beispiel 6

1998 mg Rilmakalim-Hemihydrat und 2,0 mg Soja-Lecithin S100 werden in einem Ethanol/Wasser-Gemisch (50 % G/G) klar gelöst und bei 100°C unter inerten Bedingungen (N₂) in einer Sprühtrocknungsapparatur sprühgetrocknet. Das Produkt fällt dabei als feines, weißes Pulver an. Das Pulver wird in eine für Dosieraerosole vorgeschriebene Alumonobloc-Dose zu je 100 mg eindosiert, die Dose wird mit einem Dosierventil verschlossen und anschließend mit 10 g R 227 über das Dosierventil befüllt. Nach der Füllung entsteht sofort eine feine, homogene Suspension primärer Arzneistoffteilchen in R 227. Ein Sprühstoß des nach diesem Verfahren hergestellten Inhalationsaerosols (Suspensionsaerosols) enthält pro Applikation 100 µl Suspension, enthaltend 1000 µg Arzneistoff.

### Beispiel 7

1998 mg Icatibantacetat und 2,0 mg Sorbitantrioleat (Span@85) werden in einem Ethanol/Wasser-Gemisch (25 % G/G) klar gelöst und bei 100°C unter inerten Bedingungen (N₂) in einer Sprühtrocknungsapparatur sprühgetrocknet. Das Produkt fällt dabei als feines, weißes Pulver an. Das Pulver wird in eine für Dosieraerosole vorgeschriebene Alumonobloc-Dose zu je 10 mg eindosiert, die Dose wird mit einem Dosierventil verschlossen und anschließend mit 10 g R 227 über das Dosierventil befüllt. Nach der Füllung entsteht sofort eine feine, homogene Suspension primärer Arzneistoffteilchen in R 227. Ein Sprühstoß des nach diesem Verfahren hergestellten Inhalationsaerosols (Suspensionsaerosols) enthält pro Applikation 100 µl Suspension, enthaltend 100 µg Arzneistoff.

### Beispiel 8

1998 mg Icatibantacetat und 2,0 mg Ölsäure werden in einem Aceton/Wasser-Gemisch (25 % G/G) klar gelöst und bei 80 _{°} C unter inerten Bedingungen (N₂) in einer Sprühtrocknungsapparatur sprühgetrocknet. Das Produkt fällt dabei als feines, weißes Pulver an. Das Pulver wird in eine für Dosieraerosole vorgeschriebene Alumonobloc-Dose zu je 10 mg eindosiert, die Dose wird mit einem Dosierventil verschlossen und anschließend mit 10 g R 227 über das Dosierventil befüllt. Nach der Füllung entsteht sofort eine feine, homogene Suspension primärer Arzneistoffteilchen in R 227. Ein Sprühstoß des nach diesem Verfahren hergestellten Inhalationsaerosols (Suspensionsaerosols) enthält pro Applikation 100 µl Suspension, enthaltend 100 ug Arzneistoff.

### Beispiel 9

200 mg Salbutamol, 2,0 mg Soja-Lecithin S100 und 1798 mg Laktose werden in einem Ethanol/Wasser-Gemisch (25 % G/G) klar gelöst und bei 80 ° C unter inerten Bedingungen (N₂) in einer Sprühtrocknungsapparatur sprühgetrocknet. Das Produkt fällt dabei als feines, weißes Pulver an. Das Pulver wird in eine für Dosieraerosole vorgeschriebene Alumonobloc-Dose zu je 50 mg eindosiert, die Dose wird mit einem Dosierventil verschlossen und anschließend mit 10 g R 227 über das Dosierventil befüllt. Nach der Füllung entsteht sofort eine feine, homogene Suspension primärer Arzneistoffteilchen in R 227. Ein Sprühstoß des nach diesem Verfahren hergestellten Inhalationsaerosols (Suspensionsaerosols) enthält pro Applikation 100 µl Suspension, enthaltend 50 ug Arzneistoff.

### Beispiel 10

1998 mg Prednisolon und 2,0 mg Soja-Lecithin S100 werden in einem Ethanol/Wasser-Gemisch (50 % G/G) klar gelöst und bei 80 _{°} C unter inerten Bedingungen (N₂) in einer Sprühtrocknungsapparatur sprühgetrocknet. Das Produkt fällt dabei als feines, weißes Pulver an. Das Pulver wird in eine für Dosieraerosole vorgeschriebene Alumonobloc-Dose zu je 10 mg eindosiert, die Dose wird mit einem Dosierventil verschlossen und anschließend mit 10 g R 227 über das Dosierventil befüllt. Nach der Füllung entsteht sofort eine feine, homogene Suspension primärer Arzneistoffteilchen in R 227. Ein Sprühstoß des nach diesem Verfahren hergestellten Inhalationsaerosols (Suspensionsaerosols) enthält pro Applikation 100 µl Suspension, enthaltend 100 ug Arzneistoff.

## Patentansprüche

1. Arzneistoffzubereitung in Form eines Suspensionsaerosols, dadurch gekennzeichnet, daß sie
a) ein sprühgetrocknetes Produkt bestehend aus einem Arzneistoff und einer im verflüssigten Treibmittel unlöslichen, physiologisch verträglichen, oberflächenaktiven Substanz und gegebenenfalls einem Geschmackskorrigens und gegebenenfalls einem allgemein üblichen, physiologisch verträglichen Hilfsstoff und
b) einen verflüssigbaren, hydrierten oder teilhydrierten Fluorkohlenwasserstoff als Treibmittel enthält.

2. Verfahren zur Herstellung einer Arzneistoffzubereitung in Form eines Suspensionsaerosols gemäß Anspruch 1, dadurch gekennzeichnet, daß man den Arzneistoff, die physiologisch verträgliche, oberflächenaktive Substanz und gegebenenfalls ein Geschmackskorrigens und gegebenenfalls weitere, allgemein übliche, physiologisch verträgliche Hilfsstoffe in einem geeigneten Lösungsmittel löst, die erhaltene Lösung einer Sprühtrocknung unterwirft, das sprühgetrocknete Produkt in eine Druckgaspackung eindosiert, diese mit einem Dosierventil verschließt und die zur Bildung des Suspensionsaerosols erforderliche Menge des verflüssigbaren, teilhydrierten oder hydrierten Fluorkohlenwasserstoffs zudosiert.

3. Arzneistoffzubereitung gemäß Anspruch 1, dadurch gekennzeichnet, daß sie lokal auf der Lunge wirksame, zur Inhalation geeignete Arzneistoffe zur Behandlung von Atemwegserkrankungen oder Asthma enthält.

4. Arzneistoffzubereitung gemäß Anspruch 1, dadurch gekennzeichnet, daß sie als Arzneistoff ein Peptid oder Protein oder deren physiologisch verträgliche Salze enthält.

5. Arzneistoffzubereitung gemäß Anspruch 1, dadurch gekennzeichnet, daß sie Icatibant oder dessen physiologisch verträgliches Salz enthält.

6. Arzneistoffzubereitung gemäß Anspruch 1, dadurch gekennzeichnet, daß die oberflächenaktive Substanz Sorbitantrioleat, Ölsäure oder Lecithin ist.

7. Arzneistoffzubereitung gemäß Anspruch 1, dadurch gekennzeichnet, daß das Treibmittel Heptafluorpropan ist.

8. Arzneistoffzubereitung gemäß Anspruch 1, dadurch gekennzeichnet, daß das Geschmackskorrigens ein Süßstoff und/oder ein ätherisches Öl ist und die weiteren Hilfsstoffe ausgewählt sind aus der Gruppe der Zucker und Zuckeralkohole.
